# EUROPEAN PATENT APPLICATION

(11) **EP 3 736 320 A1**
(43) Date of publication of application: **11.11.2020**
(21) Application number: 19173311.2
(22) Date of filing: 08.05.2019
(51) Int. Cl.: C11D 17/06, C11D 11/00, C11D 3/00, C11D 3/37, C11D 3/30

(54) **PARTICLES FOR THROUGH THE WASH LAUNDRY SOFTENING**

(71) Applicant: The Procter & Gamble Company, Cincinnati, OH 45202 (US)
(72) Inventor: PANANDIKER, Rajan Keshav, Cincinnati, Ohio 45202 (US); MENKHAUS, Julie Ann, Cincinnati, Ohio 45202 (US); JOHNSON, Lenae Virginia, Cincinnati, Ohio 4502 (US)
(74) Representative: P&G Patent Belgium UK

(57) **Abstract**

A particulate composition for providing a softening benefit to an article of clothing. The composition includes a water soluble or water dispersible carrier, a cationic polymer, and a fatty amine.

## Description

### FIELD OF THE INVENTION

Through the wash laundry softening additive.

### BACKGROUND OF THE INVENTION

Consumers continually express interest is products that can simplify the processes they use to launder clothes, help them reduce the amount of time they spend dealing with dirty laundry, and help them achieve high levels of cleanliness and softness for their family's clothing. Cleaning and softening of laundry presently requires consumers to dose two products to either different compartments or parts of the washing machine or to dose one product to the washing machine and one product to the dyer.

There are three basic steps to laundering fabric: washing, rinsing, and drying. The washing step typically employs water and detergent composition comprising anionic surfactant, along with other active agents that are compatible with anionic surfactants in the unused product form and in the wash liquor formed during the washing step. After washing, the laundry is rinsed one or more times as part of the rinsing step.

Presently, laundry softening is most often and practically accomplished during the rinsing step with a liquid softening composition that is separate from the detergent composition or during the drying step with a sheet releasably carrying solid softening composition. To apply liquid softening composition to the laundry in the washing machine, the liquid softening composition is introduced to the laundry during the rinsing step. The liquid softening composition may be automatically introduced into the rinse from a compartment that keeps the liquid softening composition separate from the washing composition. The compartment may be part of the agitator, if present, or another part of the washing machine that can be used to dispense the liquid softening composition into the drum. This is often referred to as softening through the rinse. Softening through the rinse requires the consumer to dose the detergent composition and the softening composition to different locations of the washing machine, which is inconvenient. Laundry softening can also be accomplished during the drying step using fabric softening sheets. With either of these approaches to cleaning and softening, cleaning is performed separately from softening.

Consumers find it inconvenient to have to dispense multiple products to different locations, whether the locations are part of the washing machine or the locations are distributed between the washing machine and the dryer. What the consumer would like is to be able to dose the detergent composition and the softening composition to a single location.

Unfortunately, liquid detergent compositions tend to be incompatible with softening compositions. Liquid detergent compositions comprise anionic surfactants to help clean the clothing. Softening compositions typically comprise cationic surfactants to soften the clothing. When combined as liquids in a single package, the anionic surfactant and cationic surfactant can combine and form a solid precipitate. This results in a problem with stability of the combination when packaged together in a liquid form or together in a wash liquor and a decrease in cleaning performance as compared to the detergent composition in absence of the softening composition. This incompatibility problem is among the reasons that detergent compositions and fabric softening compositions are dosed and applied separate from one another. Liquid fabric softening compositions packaged separately from detergent compositions may not be preferred by some consumers due to the inconvenience of dosing the composition to the washing machine, perceived messiness, and the texture of the product.

With these limitations in mind, there is a continuing unaddressed need for a solid form through the wash fabric softening composition that can be dispensed by the consumer together with the laundry detergent to providing softening through the wash during the washing step.

### SUMMARY OF THE INVENTION

A composition comprising a plurality of particles, said plurality of particles comprising:
25% to 94% by weight a water soluble or water dispersible carrier;
0.5% to 10% by weight a cationic polymer; and
5% to 45% by weight a fatty amine of the structure 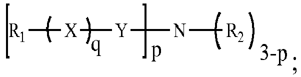
   wherein each R₁ is independently selected from the group consisting of C₈-C₃₂ alkyl, C₈-C₃₂ substituted alkyl, C₆-C₃₂ aryl, C₅-C₃₂ substituted aryl, C₆-C₃₂ alkylaryl, C₆-C₃₂ substituted alkylaryl;
   X is a 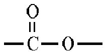 group or a 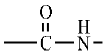 group;
   Y is an alkylene radical having 1-6 carbon atoms;
   N is a nitrogen atom;
   R₂ is independently selected from the group consisting of H, C₁-C₆ alkyl, hyroxyalkyl and polyhydroxyalkyl;
   q is 0 or 1;
   p is an integer from 1-3; and
   wherein said plurality of particles comprises individual particles, wherein said individual particles each have a mass from about 1 mg to about 1 g.

### DETAILED DESCRIPTION OF THE INVENTION

The composition described herein can provide for a through the wash fabric softening composition that is convenient for the consumer to dose to the washing machine. The through the wash fabric softening composition can be provided in a composition comprising a plurality of particles. The plurality of particles can be provided in a package that is separate from the package of detergent composition. Having the softening composition as a plurality of particles in a package separate from the package of detergent composition can be beneficial since it allows the consumer to select the amount of softening composition independent of the amount of detergent composition used. This can give the consumer the opportunity to customize the amount of softening composition used and thereby the amount of softening benefit they achieve, which is a highly valuable consumer benefit.

Particulate products, especially particulates that are not dusty, are preferred by many consumers. Particulate products can be easily dosed by consumers from a package directly into the washing machine or into a dosing compartment on the washing machine. Or the consumer can dose from the package into a dosing cup that optionally provides one or more dosing indicia and then dose the particulates into a dosing compartment on the washing machine or directly to the drum. For products in which a dosing cup is employed, particulate products tend to be less messy than liquid products.

The plurality of particles of the fabric softening composition can comprise about 25% to about 94% by weight a water soluble or water dispersible carrier, about 0.5% to about 10% by weight cationic polymer, and about 5% to about 45% by weight fatty amine. Individual particles constituting the plurality of particles can have a mass from about 1 mg to about 1 g. The fatty amine and cationic polymer can be dispersed in the carrier. The carrier carries the fatty amine and cationic polymer to the washing machine. The plurality of particles is dissolved into the wash liquor. The cationic polymer is deposited onto the fibers of the fabric and promotes deposition of the quaternary ammonium compound onto the fabric. And, the fatty amine is deposited from the wash liquor onto the fibers of the fabric. The cationic polymer and fatty amine deposited on the fibers provides the consumer with a feeling of softness.

Optionally, the plurality of particles can further comprise fatty acid. The fatty amine, cationic polymer, and fatty acid can be dispersed in the carrier.

### Water Soluble or Water Dispersible Carrier

The plurality of particles can comprise a water soluble carrier or water dispersible carrier. Upon dissolution or dispersion of the carrier, the fabric care benefit agents are dispersed into the wash liquor.

The water soluble or water dispersible carrier can be a material that is soluble or dispersible in a wash liquor within a short period of time, for instance less than about 10 minutes. The water soluble or water dispersible carrier can be selected from the group consisting of water soluble inorganic alkali metal salt, water-soluble alkaline earth metal salt, water-soluble organic alkali metal salt, water-soluble organic alkaline earth metal salt, water soluble carbohydrate, water-soluble silicate, water soluble urea, and any combination thereof.

The water soluble carrier can be a water soluble polymer. Water soluble polymers can be selected from the group consisting of polyvinyl alcohols (PVA), modified PVAs; polyvinyl pyrrolidone; PVA copolymers such as PVA/polyvinyl pyrrolidone and PVA/ polyvinyl amine; partially hydrolyzed polyvinyl acetate; polyalkylene oxides such as polyethylene oxide; polyethylene glycols; acrylamide; acrylic acid; cellulose, alkyl cellulosics such as methyl cellulose, ethyl cellulose and propyl cellulose; cellulose ethers; cellulose esters; cellulose amides; polyvinyl acetates; polycarboxylic acids and salts; polyaminoacids or peptides; polyamides; polyacrylamide; copolymers of maleic/acrylic acids; polysaccharides including starch, modified starch; gelatin; alginates; xyloglucans, other hemicellulosic polysaccharides including xylan, glucuronoxylan, arabinoxylan, mannan, glucomannan and galactoglucomannan; and natural gums such as pectin, xanthan, and carrageenan, locus bean, arabic, tragacanth; and combinations thereof. In one embodiment the polymer comprises polyacrylates, especially sulfonated polyacrylates and water-soluble acrylate copolymers; and alkylhydroxy cellulosics such as methylcellulose, carboxymethylcellulose sodium, modified carboxy-methylcellulose, dextrin, ethylcellulose, propylcellulose, hydroxyethyl cellulose, hydroxypropyl methylcellulose, maltodextrin, polymethacrylates. In yet another embodiment, the water soluble polymer can be selected from the group consisting of PVA; PVA copolymers; hydroxypropyl methyl cellulose (HPMC); and mixtures thereof.

The water soluble carrier can be selected from the group consisting of polyvinyl alcohol, modified polyvinyl alcohol, polyvinyl pyrrolidone, polyvinyl alcohol/polyvinyl pyrrolidone, polyvinyl alcohol/polyvinyl amine, partially hydrolyzed polyvinyl acetate, polyalkylene oxide, polyethylene glycol, acrylamide, acrylic acid, cellulose, alkyl cellulosics, methyl cellulose, ethyl cellulose, propyl cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides, starch, modified starch, gelatin, alginates, xyloglucans, hemicellulosic polysaccharides, xylan, glucuronoxylan, arabinoxylan, mannan, glucomannan, galactoglucomannan, natural gums, pectin, xanthan, carrageenan, locus bean, arabic, tragacanth, polyacrylates, sulfonated polyacrylates, water-soluble acrylate copolymers, alkylhydroxy cellulosics, methylcellulose, carboxymethylcellulose sodium, modified carboxy-methylcellulose, dextrin, ethylcellulose, propylcellulose, hydroxyethyl cellulose, hydroxypropyl methylcellulose, maltodextrin, polymethacrylates, polyvinyl alcohol copolymers, hydroxypropyl methyl cellulose, and mixtures thereof.

The water soluble carrier can be selected from the group consisting of polyethylene glycol, sodium acetate, sodium bicarbonate, sodium chloride, sodium silicate, polypropylene glycol polyoxoalkylene, polyethylene glycol fatty acid ester, polyethylene glycol ether, sodium sulfate, starch, and mixtures thereof.

The water soluble carrier can be polyethylene glycol (PEG). PEG can be a convenient material to employ to make individual particles because it can be sufficiently water soluble to dissolve during a wash cycle when the particles have the range of mass disclosed herein. Further, PEG can be easily processed as melt. The onset of melt temperature of PEG can vary as a function of molecular weight of the PEG. The plurality of particles can comprise about 25% to about 94% by weight PEG having a weight average molecular weight from about 2000 to about 13000. PEG has a relatively low cost, may be formed into many different shapes and sizes, and dissolves well in water. PEG comes in various weight average molecular weights. A suitable weight average molecular weight range of PEG includes from about 2,000 to about 13,000, alternatively from about 4,000 to about 13,000, alternatively from about 4,000 to about 12,000, alternatively from about 4,000 to about 11,000, alternatively from about 5,000 to about 11,000, alternatively from about 6,000 to about 10,000, alternatively from about 7,000 to about 9,000, alternatively combinations thereof. PEG is available from BASF, for example PLURIOL E 8000 (which has a weight average molecular weight of 9000 even though 8000 is in the product name), or other PLURIOL product.

The plurality of particles can comprise about 25% to about 94% by weight of the plurality of particles of PEG. Optionally, the particles can comprise from about 35% to about 94%, optionally from about 50% to about 94%, optionally combinations thereof and any whole percentages or ranges of whole percentages within any of the aforementioned ranges, of PEG by weight of the plurality of particles.

The water soluble carrier can comprise a material selected from the group consisting of: a polyalkylene polymer of formula H-(C₂H₄O)ₓ-(CH(CH₃)CH₂O)_{y}-(C₂H₄O)_{z}-OH wherein x is from about 50 to about 300, y is from about 20 to about 100, and z is from about 10 to about 200; a polyethylene glycol fatty acid ester of formula (C₂H₄O)_{q}-C(O)O-(CH₂)ᵣ-CH₃ wherein q is from about 20 to about 200 and r is from about 10 to about 30; a polyethylene glycol fatty alcohol ether of formula HO-(C₂H₄O)ₛ-(CH₂)ₜ)-CH₃ wherein s is from about 30 to about 250 and t is from about 10 to about 30; and mixtures thereof. The polyalkylene polymer of formula H-(C₂H₄O)ₓ-(CH(CH₃)CH₂O)_{y}-(C₂H₄O)_{z}-OH wherein x is from about 50 to about 300, y is from about 20 to about 100, and z is from about 10 to about 200, can be a block copolymer or random copolymer.

The water soluble carrier can comprise: polyethylene glycol; a polyalkylene polymer of formula H-(C₂H₄O)ₓ-(CH(CH₃)CH₂O)_{y}-(C₂H₄O)_{z}-OH wherein x is from about 50 to about 300; y is from about 20 to about 100, and z is from about 10 to about 200; a polyethylene glycol fatty acid ester of formula (C₂H₄O)_{q}-C(O)O-(CH₂)ᵣ-CH₃ wherein q is from about 20 to about 200 and r is from about 10 to about 30; and a polyethylene glycol fatty alcohol ether of formula HO-(C₂H₄O)ₛ-(CH₂)ₜ)-CH₃ wherein s is from about 30 to about 250 and t is from about 10 to about 30.

The water soluble carrier can comprise from about 20% to about 80% by weight of the particles of polyalkylene polymer of formula H-(C₂H₄O)ₓ-(CH(CH₃)CH₂O)_{y}-(C₂H₄O)_{z}-OH wherein x is from about 50 to about 300; y is from about 20 to about 100, and z is from about 10 to about 200.

The water soluble carrier can comprise from about 1% to about 20% by weight of the particles polyethylene glycol fatty acid ester of formula (C₂H₄O)_{q}-C(O)O-(CH₂)ᵣ-CH₃ wherein q is from about 20 to about 200 and r is from about 10 to about 30.

The water soluble carrier can comprise from about 1% to about 10% by weight of the plurality particles of polyethylene glycol fatty alcohol ether of formula HO-(C₂H₄O)ₛ-(CH₂)ₜ)-CH₃ wherein s is from about 30 to about 250 and t is from about 10 to about 30.

The water soluble carrier can be selected from the group consisting of: a polyalkylene polymer of formula H-(C₂H₄O)ₓ-(CH(CH₃)CH₂O)_{y}-(C₂H₄O)_{z}-OH wherein x is from about 50 to about 300, y is from about 20 to about 100, and z is from about 10 to about 200; a polyethylene glycol fatty acid ester of formula (C₂H₄O)_{q}-C(O)O-(CH₂)ᵣ-CH₃ wherein q is from about 20 to about 200 and r is from about 10 to about 30; a polyethylene glycol fatty alcohol ether of formula HO-(C₂H₄O)ₛ-(CH₂)ₜ)-CH₃ wherein s is from about 30 to about 250 and t is from about 10 to about 30; C8-C22 alkyl polyalkoxylate comprising more than about 40 alkoxylate units; polyethylene glycol having a weight average molecular weight from about 2000 to about 15000; EO/PO/EO block copolymer; PO/EO/PO block copolymer; EO/PO block copolymer; PO/EO block copolymer; polypropylene glycol; ethoxylated nonionic surfactant having a degree of ethoxylation greater than about 30; polyvinyl alcohol; polyalkylene glycol having a weight average molecular weight from about 2000 to about 15000; and mixtures thereof.

The plurality of particles of the fabric softening composition can comprise about 25% to about 94% by weight a water soluble or water dispersible carrier. Optionally, the plurality of particles of the fabric softening composition can comprise about 30% to about 94% by weight, optionally about 35% to about 85%, optionally about 35% to about 94%, a water soluble or water dispersible carrier.

### Fatty Amine

The fatty amine can be of the structure: 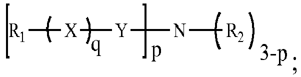
wherein each R₁ is independently selected from the group consisting of C₈-C₃₂ alkyl, C₈-C₃₂ substituted alkyl, C₆-C₃₂ aryl, C₅-C₃₂ substituted aryl, C₆-C₃₂ alkylaryl, C₆-C₃₂ substituted alkylaryl;
X is a 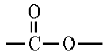 group or a 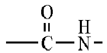 group;
Y is an alkylene radical having 1-6 carbon atoms; N is a nitrogen atom; R₂ is independently selected from the group consisting of H, C₁-C₆ alkyl, hyroxyalkyl and polyhydroxyalkyl; q is 0 or 1; and p is an integer from 1-3.

Optionally, each R₁ is independently C₁₀-C₂₂ alkyl or C₈-C₂₂ substituted alkyl;
X is the 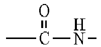 group; Y is an alkylene radical having 2-4 carbon atoms; R₂ is independently H or C₁-C₆ alkyl; q is 1; and p is 1.

Optionally, each R₁ is independently C₁₀-C₂₂ alkyl and R₂ is a methyl group
The fatty amine can be of the structure 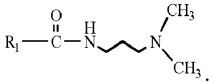 Optionally, R₁ is a C₁₂-C₂₂ alkyl; X is a 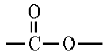 group; Y is an alkylene radical with 2-4 carbon atoms; R₂ is independently selected from the group consisting of H, C₁-C₆ alkyl, hyroxyalkyl and polyhydroxyalkyl groups; q=1; and p=1 to 3.

The fatty amine can have a structure selected from the group consisting of: 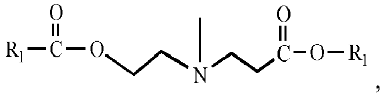 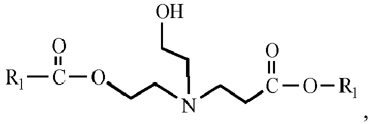 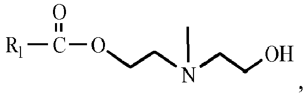 and 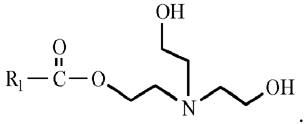 Optionally each R₁ is independently C₁₀-C₁₈ alkyl; Y is an alkylene radical with 2-4 carbon atoms, R₂ is independently selected from the group consisting of H, C₁-C₆ alkyl, hyroxyalkyl and polyhydroxyalkyl groups, p is an integer from 1-3 and q =0
Optionally, R₂ is a methyl or hydydroxyethyl group;

The fatty amine can have a structure selected from the group consisting of:

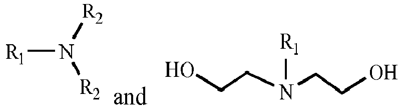

The fatty amine can be selected from the group consisting of: fatty esters of bis-(2-hydroxypropyl)-methylamine, bis-(hydroxyethyl)-methyl amine, bis-(hydroxyethyl)-isopropyl amine and triethanolamine with at least one fatty acid comprising C₁₂-C₂₂ alkyl chain; N,N-bis-(stearoyl-2-hydroxypropyl)-N-methylamine; N,N-bis(stearoyl-oxy-ethyl)-N-methyl amine; N, N-bis(stearoyl-oxy-ethyl)-N-hydroxyethylamine; N-(stearoyl-oxy-ethyl)-N, N-dimethyl amine; N-(stearoyl-oxy-ethyl)-N, N-hydroxyethylamine; N,N,N-tris(stearoyl-oxy-ethyl)-amine; stearylamidopropyl dimethyl amine; cocoamidopropyl dimethylamine; behenylamidopropyl dimethyl amine; stearylamine; distearylamine; tristearylamine; and N, N-distearyl N-methylamine;

### Fatty Acid

The plurality of particles can further comprise a fatty acid. The fatty acid can be provided at a molar ratio of the fatty amine to fatty acid from about 1:3 to about 3:1.

The term fatty acid is used herein in the broadest sense to include unprotonated or protonated forms of a fatty acid. The fatty acid may include those containing from 12 to 25, from 13 to 22, or even from 16 to 20, total carbon atoms, with the fatty moiety containing from 10 to 22, from 12 to 18, or even from 14 (mid-cut) to 18 carbon atoms. Mixtures of fatty acids from different fat sources can be used.

The cis/trans ratio for the unsaturated fatty acids may be important, with the cis/trans ratio (of the C18:1 material) being from at least 1:1, at least 3:1, from 4:1 or even from 9:1 or higher. Branched fatty acids such as isostearic acid are also suitable since they may be more stable with respect to oxidation and the resulting degradation of color and odor quality. The fatty acid may have an iodine value from 0 to 140, from 50 to 120 or even from 85 to 105.

The plurality of particles can comprise from about 1% to about 40% by weight fatty acid. The fatty acid can be selected from the group consisting of, a saturated fatty acids, unsaturated fatty acid, and mixtures thereof. The fatty acid can be a blend of saturated fatty acids, a blend of unsaturated fatty acids, and mixtures thereof. The fatty acid can be substituted or unsubstituted. The fatty acid can be provided with the quaternary ammonium compound. The fatty acid can have an Iodine Value of zero.

The fatty acid can be selected from the group consisting of stearic acid, palmitic acid, coconut oil, palm kernel oil, stearic acid palmitic acid blend, oleic acid, vegetable oil, partially hydrogenated vegetable oil, and mixtures thereof. The fatty acid can be selected from the group consisting of stearic acid, aryl sulfonic acid, cumenesulfonic acid, xylene sulfonic acid, toluene sulfonic acid, alkenyl succinic acid, octyl succinic acid, dodecenylsuccinic acid, C₁₂-C₁₈ fatty acid, oleic acid, lauric acid myristic acid, and combinations thereof.

The fatty acid can be Stearic acid CAS No. 57-11-4. The fatty acid can be palmitic acid CAS No. 57-10-3. The fatty acid can be a blend of stearic acid and coconut oil.

The fatty acid can be C12 to C22 fatty acid. C12 to C22 fatty acid can have tallow or vegetable origin, can be saturated or unsaturated, can be substituted or unsubstituted.

Without being bound by theory, fatty acid may help as a processing aid for uniformly mixing the formulation components of the individual particles constituting the plurality of particles.

### Cationic Polymer

The plurality of particles can comprise a cationic polymer. Cationic polymers can provide the benefit of a deposition aid that helps to deposit onto the fabric quaternary ammonium compound and possibly some other benefit agents that are contained in the particles.

The plurality of particles can comprise about 0.5% to about 10% by weight cationic polymer. Optionally, the plurality of particles can comprise about 0.5% to about 5% by weight cationic polymer, or even about 1% to about 5% by weight, or even about 2% to about 4% by weight cationic polymer, or even about 3% by weight cationic polymer. Without being bound by theory, it is thought that the cleaning performance of laundry detergent in the wash decreases with increasing levels of cationic polymer in the particles and acceptable cleaning performance of the detergent can be maintained within the aforesaid ranges.

The cationic polymer can have a cationic charge density more than about 0.05 meq/g (meq meaning milliequivalents), to 23 meq/g , preferably from about 0.1 meq/g to about 4 meq/g. even more preferably from about 0.1 meq/g to about 2 meq/g and most preferably from 0.1meq/g to about 1 meq/g.

The above referenced cationic charge densities can be at the pH of intended use, which can be a pH from about 3 to about 9, optionally about 4 to about 9.

Cationic charge density of a polymer refers to the ratio of the number of positive charges on the polymer to the molecular weight of the polymer. Charge density is calculated by dividing the number of net charges per repeating unit by the molecular weight of the repeating unit. The positive charges may be located on the backbone of the polymers and/or the side chains of polymers. The average molecular weight of such suitable cationic polymers can generally be between about 10,000 and about 10 million, or even between about 50,000 and about 5 million, or even between about 100,000 and about 3 million.

Non-limiting examples of cationic polymers are cationic or amphoteric, polysaccharides, proteins and synthetic polymers. Cationic polysaccharides include cationic cellulose derivatives, cationic guar gum derivatives, chitosan and its derivatives and cationic starches. Cationic polysaccharides have a molecular weight from about 1,000 to about 2 million, preferably from about 100,000 to about 800,000. Suitable cationic polysaccharides include cationic cellulose ethers, particularly cationic hydroxyethylcellulose and cationic hydroxypropylcellulose. Particularly preferred are cationic cellulosic polymers with substituted anhydroglucose units that correspond to the general Structural Formula as follows: 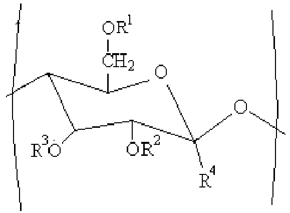
Wherein R¹, R², R³ are each independently selected from H, CH₃, C₈₋₂₄ alkyl (linear or branched), 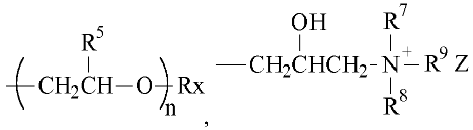 or mixtures thereof;
   R⁴ is H,
   n is from about 1 to about 10;
   Rx is seclected from the group consisting of H, CH₃, C₈₋₂₄ alkyl (linear or branched), 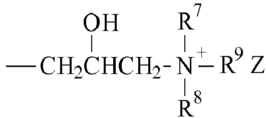 or mixtures thereof, wherein Z is a water soluble anion, preferably a chlorine ion and/or a bromine ion; R⁵ is H, CH₃, CH₂CH₃, or mixtures thereof; R⁷ is CH₃, CH₂CH₃, a phenyl group, a C₈₋₂₄ alkyl group (linear or branched), or mixture thereof; and
   R⁸ and R⁹ are each independently CH₃, CH₂CH₃, phenyl, or mixtures thereof:
With the provisio that at least one of R¹, R², R³ groups per anhydroglucose unit is 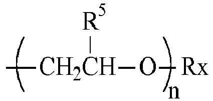 and each polymer has at least one 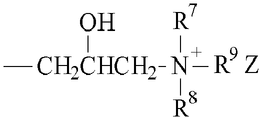 group.

The charge density of the cationic celluloses herein (as defined by the number of cationic charges per 100 anhydroglucose units) is preferably from about 0.5 % to about 60%, more preferably from about 1% to about 20%, and most preferably from about 2% to about 10%.

Alkyl substitution on the anhydroglucose rings of the polymer ranges from about 0.01% to 5% per glucose unit, more preferably from about 0.05% to 2% per glucose unit, of the polymeric material.

The cationic cellulose may lightly cross-linked with a dialdehyde such as glyoxyl to prevent forming lumps, nodules or other agglomerations when added to water at ambient temperatures.

Examples of cationic hydroxyalkyl cellulose include those with the INCI name Polyquaternium10 such as those sold under the trade names UCARE Polymer JR 30M, JR 400, JR 125, LR 400 and LK 400, Polymer PK polymers; Polyquaternium 67 such as those sold under the trade name SOFTCAT SK TM, all of which are marketed by Dow Chemicals, Midlad MI, and Polyquaternium 4 such as those sold under the trade name CELQUAT H200 and CELQUAT L-200 available from National Starch and Chemical Company, Bridgewater, NJ. Other suitable polysaccharides include hydroxyethyl cellulose or hydoxypropylcellulose quaternized with glycidyl C₁₂-C₂₂ alkyl dimethyl ammonium chloride. Examples of such polysaccharides include the polymers with the INCI names Polyquaternium 24 such as those sold under the trade name QUATERNIUM LM 200 by Dow Chemicals of Midland, MI. Cationic starches refer to starch that has been chemically modified to provide the starch with a net positive charge in aqueous solution at pH 3. This chemical modification includes, but is not limited to, the addition of amino and/or ammonium group(s) into the starch molecules. Non-limiting examples of these ammonium groups may include substituents such as trimethylhydroxypropyl ammonium chloride, dimethylstearylhydroxypropyl ammonium chloride, or dimethyldodecylhydroxypropyl ammonium chloride. The source of starch before chemical modification can be chosen from a variety of sources including tubers, legumes, cereal, and grains. Non-limiting examples of this source of starch may include corn starch, wheat starch, rice starch, waxy corn starch, oat starch, cassaya starch, waxy barley, waxy rice starch, glutenous rice starch, sweet rice starch, amioca, potato starch, tapioca starch, oat starch, sago starch, sweet rice, or mixtures thereof. Nonlimiting examples of cationic starches include cationic maize starch, cationic tapioca, cationic potato starch, or mixtures thereof. The cationic starches may comprise amylase, amylopectin, or maltodextrin. The cationic starch may comprise one or more additional modifications. For example, these modifications may include cross-linking, stabilization reactions, phophorylations, hydrolyzations, cross-linking. Stabilization reactions may include alkylation and esterification. Suitable cationic starches for use in the present compositions are commercially-available from Cerestar under the trade name C*BOND and from National Starch and Chemical Company under the trade name CATO 2A. Cationic galactomannans include cationic guar gums or cationic locust bean gum. An example of a cationic guar gum is a quaternary ammonium derivative of hydroxypropyl guar such as those sold under the trade name JAGUAR C13 and JAGUAR Excel available from Rhodia, Inc of Cranbury NJ and N-HANCE by Aqualon, Wilmington, DE

Other suitable cationic polymers for use in the plurality of particles include polysaccharide polymers, cationic guar gum derivatives, quaternary nitrogen-containing cellulose ethers, synthetic polymers, copolymers of etherified cellulose, guar and starch. When used, the cationic polymers herein are either soluble in the composition used to form the particles or are soluble in a complex coacervate phase in the composition from which the particles are formed. Suitable cationic polymers are described in U.S. Pat. Nos. 3,962,418; 3,958,581; and U.S. Publication No. 2007/0207109A1.

One group of suitable cationic polymers includes those produced by polymerization of ethylenically unsaturated monomers using a suitable initiator or catalyst, such as those disclosed in WO 00/56849 and USPN 6,642,200. Suitable cationic polymers may be selected from the group consisting synthetic polymers made by polymerizing one or more cationic monomers selected from the group consisting of N,N-dialkylaminoalkyl acrylate, N,N-dialkylaminoalkyl methacrylate, N,N-dialkylaminoalkyl acrylamide, N,N-dialkylaminoalkylmethacrylamide, quaternized N, N dialkylaminoalkyl acrylate quaternized N,N-dialkylaminoalkyl methacrylate, quaternized N,N-dialkylaminoalkyl acrylamide, quaternized N,N-dialkylaminoalkylmethacrylamide, Methacryloamidopropyl-pentamethyl-1,3-propylene-2-ol-ammonium dichloride, N,N,N,N',N',N",N"-heptamethyl-N"-3-(1-oxo-2-methyl-2-propenyl)aminopropyl-9- oxo-8-azo-decane-1,4,10-triammonium trichloride, vinylamine and its derivatives, allylamine and its derivatives, vinyl imidazole, quaternized vinyl imidazole and diallyl dialkyl ammonium chloride and combinations thereof, and optionally a second monomer selected from the group consisting of acrylamide, N,N-dialkyl acrylamide, methacrylamide, N,N-dialkylmethacrylamide, C₁-C₁₂ alkyl acrylate, C₁-C₁₂ hydroxyalkyl acrylate, polyalkylene glyol acrylate, C₁-C₁₂ alkyl methacrylate, C₁-C₁₂ hydroxyalkyl methacrylate, polyalkylene glycol methacrylate, vinyl acetate, vinyl alcohol, vinyl formamide, vinyl acetamide, vinyl alkyl ether, vinyl pyridine, vinyl pyrrolidone, vinyl imidazole, vinyl caprolactam, and derivatives, acrylic acid, methacrylic acid, maleic acid, vinyl sulfonic acid, styrene sulfonic acid, acrylamidopropylmethane sulfonic acid (AMPS) and their salts. The polymer may optionally be branched or cross-linked by using branching and crosslinking monomers. Branching and crosslinking monomers include ethylene glycoldiacrylate divinylbenzene, and butadiene. A suitable polyethyleneinine useful herein is that sold under the tradename LUPASOL by BASF, AG, Ludwigshafen, Germany

In another aspect, the cationic polymer may be selected from the group consisting of cationic polysaccharide, polyethylene imine and its derivatives, poly(acrylamide-co-diallyldimethylammonium chloride), poly(acrylamide-methacrylamidopropyltrimethyl ammonium chloride), poly(acrylamide-co-N,N-dimethyl aminoethyl acrylate) and its quaternized derivatives, poly(acrylamide-co-N,N-dimethyl aminoethyl methacrylate) and its quaternized derivative, poly(hydroxyethylacrylate-co-dimethyl aminoethyl methacrylate), poly(hydroxpropylacrylate-co-dimethyl aminoethyl methacrylate), poly(hydroxpropylacrylate-co-methacrylamidopropyltrimethylammonium chloride), poly(acrylamide-co-diallyldimethylammonium chloride-co-acrylic acid), poly(acrylamide-methacrylamidopropyltrimethyl ammonium chloride-co-acrylic acid), poly(diallyldimethyl ammonium chloride), poly(vinylpyrrolidone-co-dimethylaminoethyl methacrylate), poly(ethyl methacrylate-co-quaternized dimethylaminoethyl methacrylate), poly(ethyl methacrylate-co-oleyl methacrylate-co-diethylaminoethyl methacrylate), poly(diallyldimethylammonium chloride-co-acrylic acid), poly(vinyl pyrrolidone-co-quaternized vinyl imidazole) and poly(acrylamide-co-Methacryloamidopropyl-pentamethyl-1,3-propylene-2-ol-ammonium dichloride), Suitable cationic polymers include Polyquaternium-1, Polyquaternium-5, Polyquaternium-6, Polyquaternium-7, Polyquaternium-8, Polyquaternium-10, Polyquaternium-11, Polyquaternium-14, Polyquaternium-22, Polyquaternium-28, Polyquaternium-30, Polyquaternium-32 and Polyquaternium-33, as named under the International Nomenclature for Cosmetic Ingredients.

In another aspect, the cationic polymer may comprise polyethyleneimine or a polyethyleneimine derivative. In another aspect, the cationic polymer may comprise a cationic acrylic based polymer. In a further aspect, the cationic polymer may comprise a cationic polyacrylamide. In another aspect, the cationic polymer may comprise a polymer comprising polyacrylamide and polymethacrylamidoproply trimethylammonium cation. In another aspect, the cationic polymer may comprise poly(acrylamide- N-dimethyl aminoethyl acrylate) and its quaternized derivatives. In this aspect, the cationic polymer may be that sold under the tradename SEDIPUR, available from BTC Specialty Chemicals, a BASF Group, Florham Park, N.J. In a yet further aspect, the cationic polymer may comprise poly(acrylamide-co-methacrylamidopropyltrimethyl ammonium chloride). In another aspect, the cationic polymer may comprise a non-acrylamide based polymer, such as that sold under the tradename RHEOVIS CDE, available from Ciba Specialty Chemicals, a BASF group, Florham Park, N.J., or as disclosed in USPA 2006/0252668.

In another aspect, the cationic polymer may be selected from the group consisting of cationic polysaccharides. In one aspect, the cationic polymer may be selected from the group consisting of cationic cellulose ethers, cationic galactomannan, cationic guar gum, cationic starch, and combinations thereof

Another group of suitable cationic polymers may include alkylamine-epichlorohydrin polymers which are reaction products of amines and oligoamines with epicholorohydrin, for example, those polymers listed in, for example, USPNs 6,642,200 and 6,551,986. Examples include dimethylamine-epichlorohydrin-ethylenediamine, available under the trade name CARTAFIX CB, CARTAFIX TSF, available from Clariant, Basle, Switzerland.

Another group of suitable synthetic cationic polymers may include polyamidoamine-epichlorohydrin (PAE) resins of polyalkylenepolyamine with polycarboxylic acid. The most common PAE resins are the condensation products of diethylenetriamine with adipic acid followed by a subsequent reaction with epichlorohydrin. They are available from Hercules Inc. of Wilmington DE under the trade name KYMENE from BASF AG (Ludwigshafen, Germany) under the trade name LURESIN.

The cationic polymers may contain charge neutralizing anions such that the overall polymer is neutral under ambient conditions. Non-limiting examples of suitable counter ions (in addition to anionic species generated during use) include chloride, bromide, sulfate, methylsulfate, sulfonate, methylsulfonate, carbonate, bicarbonate, formate, acetate, citrate, nitrate, and mixtures thereof.

The weight-average molecular weight of the cationic polymer may be from about 500 to about 5,000,000, or from about 1,000 to about 2,000,000, or from about 5000 to about 1,000,000 Daltons, as determined by size exclusion chromatography relative to polyethyleneoxide standards with RI detection. In one aspect, the weight-average molecular weight of the cationic polymer may be from about 100,000 to about 800,000 Daltons.

The cationic polymer can be provided in a powder form. The cationic polymer can be provided in an anhydrous state.

### Dispersing Aid

The particles can further comprise a dispersing aid. The dispersing aid can be a nonionic surfactant. The dispersing aid can be branched isotridecyl alcohol having an average of 11 EO units. The dispersing aid can be SURFONIC TDA-11, available from Huntsman Corp, Salt Lake City, UT.

### Antibacterial Active Ingredient

The particles can comprise or further comprise an antibacterial active ingredient. The antibacterial active ingredient can be 4,4' dichloro 2-hydroxydiphenyl ether (or Hydroxydichlorodiphenyl Ether). The antibacterial agent can be TINOSAN HP100, available from BASF. The antibacterial active ingredient can have the following structure:

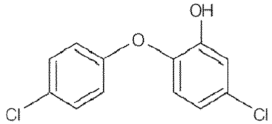

The particles can comprise from about 0.1% to about 1% by weight, or even about 0.3% to about 0.7% by weight, or even about 0.5% to about 0.6% by weight antibacterial active ingredient. The particles can comprise from about 25% to about 99.9% by weight water soluble or water dispersible carrier and about 0.1% to about 1% by weight antibacterial active ingredient, and one or more optional components at a weight percent from about 0.1% to about 75%. The particles can comprise from about 88% to about 91% by weight water soluble or water dispersible carrier (e.g. polyethylene glycol having a weight average molecular weight of 9000) and from about 0.1 to about 1% by weight antibacterial active ingredient (e.g. 4,4' dichloro 2-hydroxydiphenyl ether). The particles may be devoid of one or more of, cationic polymer, fatty amine, and fatty acid.

### Particles

The individual particles constituting the plurality of particles can have individual mass from about 1 mg to about 1 g, alternatively from about 5 mg to about 500 mg, alternatively from about 5 mg to about 200 mg, alternatively from about 10 mg to about 100 mg, alternatively from about 20 mg to about 50 mg, alternatively from about 35 mg to about 45 mg, alternatively about 38 mg. The smaller the individual particles the faster they tend to dissolve in water. The individual particles constituting the plurality of particles can have standard deviation of mass of less than about 30 mg, alternatively less than about 15 mg, alternatively less than about 5 mg, alternatively about 3 mg. The mean particle of mass within the aforesaid ranges can provide for a dispersion time in water that permits the particles to dissolve during a typical wash cycle. The standard deviation of mass within the aforesaid ranges can provide for a relatively uniform dispersion time in water. The plurality of particles can be substantially free from particles having a mass less than 10 mg. This can be practical for limiting the ability of the particles to become airborne.

The individual particles constituting the plurality of particles can have an average maximum cross sectional dimension of from about 0.05 mm to about 20 mm, optionally from about 0.5 mm to about 10 mm, optionally from about 1 mm to about 5 mm.

The individual particles may have any shape. The shape may include any basic three-dimensional shape, such as spheres, hemispheres, oblate spheres, spheroids, discs, plates, cones, truncated cones, prisms, cylinders, pyramids, noodles, rectangles, doughnuts, toroids, and the like. The shape may be formed to resemble recognizable shapes such as a heart, star, shamrock, pretzel, "smiley face" and the like. The shape may include recognizable imagery such as icons and logos including logos representative of product brands. The shapes may be uniform shapes, a combination of different shapes, or generally random shapes (such as prills).

The individual particles may have an aspect ratio that is the ratio of maximum cross-sectional dimension of the particle to the longest dimension which is perpendicular to the maximum cross-sectional dimension and entirely within the outer perimeter of the particle. The aspect ratio of a single particle, or the average aspect ratio of a plurality of particles, can be from about 1:1 to about 1000:1, optionally from about 1:1 to about 100:1, optionally from about 1:1 to about 10:1, optionally from about 1:1 to about 2:1.

A plurality of particles may collectively comprise a dose for dosing to a laundry washing machine or laundry wash basin. A single dose of the plurality of particles may comprise from about 1 g to about 50 g of particles, optionally from about 5 g to about 50 g, alternatively from about 10g to about 45 g, alternatively from about 20 g to about 40 g, alternatively combinations thereof and any whole numbers of grams or ranges of whole numbers of grams within any of the aforementioned ranges.

The plurality of particles can comprise an antioxidant. The antioxidant can help to promote stability of the color and or odor of the particles over time between production and use. The plurality of particles can comprise from about 0.01% to about 1% by weight antioxidant, optionally from about 0.001% to about 2% by weight antioxidant, optionally from about 0.01% to about 0.1% by weight antioxidant. The antioxidant can be butylated hydroxytoluene.

### Process for Treating an Article of Clothing

The plurality of particles disclosed herein enable consumers to achieve softening through the wash, in particular the wash sub-cycle. By providing softening through the wash sub-cycle, consumers only need to dose the detergent composition and the particles to a single location, for example the wash basin, prior to or shortly after the start of the washing machine. This can be more convenient to consumers than using a liquid fabric enhancer that is separately dispensed into the wash basin after the wash sub-cycle is completed, for example prior to, during, or in between rinse cycles. It can be inconvenient to use auto-dispensing features of modern upright and high efficiency machines since that requires dispensing the fabric softening composition to a location other than where detergent composition is dispensed.

The process for treating an article of clothing can comprise the steps of providing an article of clothing in a washing machine. The article of clothing is contacted during the wash sub-cycle of the washing machine with a composition comprising a plurality of particles disclosed herein. The individual particles can dissolve into water provided as part of the wash sub-cycle to form a liquor. The dissolution or dispersion of the individual particles can occur during the wash sub-cycle.

Washing machines have at least two basic sub-cycles within a cycle of operation: a wash sub-cycle and a rinse sub-cycle. The wash sub-cycle of a washing machine is the cycle on the washing machine that commences upon first filling or partially filing the wash basin with water. A main purpose of the wash sub-cycle is to remove and or loosen soil from the article of clothing and suspend that soil in the wash liquor. Typically, the wash liquor is drained at the end of the wash sub-cycle. The rinse sub-cycle of a washing machine occurs after the wash sub-cycle and has a main purpose of rinsing soil, and optionally some benefit agents provided to the wash sub-cycle from the article of clothing.

The process can optionally comprise a step of contacting the article of clothing during the wash sub-cycle with a detergent composition comprising an anionic surfactant. Most consumers provide a detergent composition to the wash basin during the wash sub-cycle. Detergent compositions can comprise anionic surfactant, and optionally other benefit agents including but not limited to perfume, bleach, brighteners, hueing dye, enzyme, and the like. During the wash sub-cycle, the benefit agents provided with the detergent composition are contacted with or applied to the article of clothing disposed in the wash basin. Typically, the benefit agents of detergent compositions are dispersed in a wash liquor of water and the benefit agents.

During the wash sub-cycle, the wash basin may be filled or at least partially filled with water. The individual particles can dissolve or disperse into the water to form a wash liquor comprising the components of the individual particles. Optionally, if a detergent composition is employed, the wash liquor can include the components of the detergent composition and the individual particles or dissolved individual particles. The plurality of particles can be placed in the wash basin of the washing machine before the article of clothing is placed in the wash basin of the washing machine. The plurality of particles can be placed in the wash basin of the washing machine after the article of clothing is placed in the wash basin of the washing machine. The plurality of particles can be placed in the wash basin prior to filling or partially filling the wash basin with water or after filling of the wash basin with water has commenced.

If a detergent composition is employed by the consumer in practicing the process of treating an article of clothing, the detergent composition and plurality of particles can be provided from separate packages. For instance, the detergent composition can be a liquid detergent composition provided from a bottle, sachet, water soluble pouch, dosing cup, dosing ball, or cartridge associated with the washing machine. The plurality of particles can be provided from a separate package, by way of non-limiting example, a carton, bottle, water soluble pouch, dosing cup, sachet, or the like. If the detergent composition is a solid form, such as a powder, water soluble fibrous substrate, water soluble sheet, water soluble film, water soluble film, water insoluble fibrous web carrying solid detergent composition, the plurality of particles can be provided with the solid form detergent composition. For instance, the plurality of particles can be provided from a container containing a mixture of the solid detergent composition and the plurality of particles. Optionally, the plurality of particles can be provided from a pouch formed of a detergent composition that is a water soluble fibrous substrate, water soluble sheet, water soluble film, water soluble film, water insoluble fibrous web carrying solid detergent composition.

### Production of Individual Particles

For a carrier that can be processed conveniently as a melt, the rotoforming process can be used. A mixture of molten carrier and the other materials constituting the particles is prepared, for instance in a batch or continuous mixing process. The plurality of particles can be produced on a rotoforming device to which the molten mixture is fed, for instance a Sandvik ROTOFORM 3000. Optionally, the individual particles can be provided with inclusions of a gas. Such occlusions of gas, for example air, can help the particles dissolve more quickly in the wash. Occlusions of gas can be provided, by way of nonlimiting example, by injecting gas into the molten precursor material and milling the mixture.

### Onset of Melt Test Method

Onset of melt is determined using the Onset of Melt Test Method as follows. Differential Scanning Calorimetry (DSC) is used to quantify the temperature at which the onset of melt occurs for the peak melt transition of any given composition of individual particles to be tested. The melt temperature measurements are made using a high quality DSC instrument with accompanying software and nitrogen purge capability, such as TA Instruments' model Discovery DSC (TA Instruments Inc. / Waters Corporation, New Castle, Delaware, U.S.A.). A calibration check is conducted using an Indium standard sample. The DSC instrument is considered suitable to conduct the test if the onset of melt temperature measured for the Indium standard sample is within the range of 156.3 - 157.3 °C.

A plurality of particles of the test composition are examined to identify individual particles which comprise a first set of particle versus those which comprise a second set of particle, and those that comprise any additional number of sets which may be present. The process of examining a plurality of particles to achieve such set identifications may include many approaches, including the examination and comparison of individual particles by visual inspection, examination and comparison of individual particles based on chemical makeup, and by chemical testing to determine the presence or absence of fatty amine, cationic polymer, or fatty acid in the individual particles. Test compositions are to be tested on a per set basis (i.e., by physically separating individual particles according to their set, thus creating internally uniform samples wherein each sample comprises a single set of individual particles). These samples are used to test a group of individual particles from each set separately from particles of other sets. The results measured for each set of individual particles are reported separately (i.e. on a per set basis).

A uniform test sample is prepared by obtaining at least 5g of individual particles, which are then pulverised via milling into powder form using an analytical milling device, such as the IKA basic analytical mill model A11 B S1 (IKA-Werke GmbH & Co. KG, Staufen im Breisgau, Germany). The milled sample is subsequently sieved through a clean stainless steel sieve with sieve mesh size openings of nominally 1mm in diameter (e.g. number 18 mesh size). For each sample to be tested, at least two replicate samples are independently milled and measured. A sample of the milled material weighing approximately 5 mg is placed into the bottom of a hermetic aluminium DSC sample pan, and the sample is spread out to cover the base of the pan. A hermetic aluminium lid is placed on the sample pan, and the lid is sealed with a sample encapsulating press to prevent evaporation or weight loss during the measurement process. The DSC measurements are conducted relative to a reference standard. An empty aluminum DSC sample pan used as the reference standard, in order to measure the delta in heat adsorption of the sample-containing pan versus the empty reference pan.

The DSC instrument is set up to analyze samples using the following cycle configuration selections: Sample Purge Gas is nitrogen set at 50 mL/min; Sampling Interval is set at 0.1 s/point; Equilibrate is set at -20.00 °C; Isothermal Hold is set at 1 min. Data is collected during a single heating cycle using the settings: Ramp is set at 10.00 °C/min to 90.00 °C; and Isothermal Hold is set at 90.00 °C for 1 min. A sealed sample pan containing a replicate test sample is carefully loaded into the instrument, as is an empty reference pan. The DSC analysis cycle specified above is conducted and the output data is assessed. The data acquired during the DSC heating cycle is typically plotted with Temperature on the X-axis (in °C) and Heat Flow normalized to sample weight (in W/g) on the Y-axis, such that melting points appear as downward (endothermic) peaks since they absorb energy.

A melt transition onset temperature is the temperature at which a deflection is first observed from the baseline previously established for the melt temperature of interest. The Peak Melt temperature is the specific temperature that requires the largest observed differential energy to transition the sample from a solid phase to a melt phase, during the specified DSC heating cycle. For the purpose of this invention, the Onset of Melt temperature is defined as the melt transition onset temperature for the Peak Melt temperature. Additional general information on the DSC technique may be found in the industry standard method ASTM D3418-03 - Transition Temperatures of Polymers by DSC.

Using the DSC instrument software, two points are manually defined as the "Start and Stop Integration" baseline limits. The two points selected are on flat regions of the baseline to the left and right sides, respectively, of the melt transition peak detected. This defined area is then used to determine the peak temperature (T) which can be used to report the Peak Melt Temperature. The Onset of Melt temperature for the Peak Melt temperature is then identified by the instrument software.

The Onset of Melt temperature reported is the average result (in °C) from the replicate samples of that set of particle.

### Particle Dissolution and Coefficient of Friction Testing

Specimens of particles were prepared to determine the particle dissolution time in water. The specimens were prepared by providing polyethylene glycol having a weight average molecular weight of 9000 in a 0.47 L glass jar and placing the cup of material in an oven having a temperature of 80 C overnight to melt. A speed mix cup (Max 100 SPEEDMIX Cup) of the fatty amine, and fatty acid if employed, was placed in the same oven for four hours to melt. After melting the contents of the 100 SPEEDMIX cup were thoroughly mixed by swirling the contents. The melted polyethylene glycol was then added to the speed mix cup. Then the cationic polymer, if employed, was added to the speed mix cup. The speed cup of materials was placed into a SPEEDMIXER DAC 150 FVC-K (FLAK TEK Inc.) for 60 seconds at 3500 revolutions per minute. The mixture was then immediately poured onto a rubber mold that was initially at room temperature and spread with a spatula into depressions in the rubber mold. The mixture hardened in the depressions of the rubber mold to form the particles. The hardened particles were removed from the rubber mold. The mold shape was an oblate hemisphere having a diameter of 5.0 mm and a height of 2.5. Particle dissolution time testing was performed as follows. 500 mL of 25 C, 137 parts per million hardness was placed into a 600 mL beaker. A 41 mm x 8 mm stir bar was placed in the beaker. The beaker was then place on a stir plate and stirred at 400 revolutions per minute. 0.6 g of TIDE FREE detergent, available from THE PROCTER & GAMBLE COMPANY, was added and mixed for 30 seconds. Five particles, each having a mass of 38 mg +/- 3 mg, were simultaneously added to the beaker and a timer was started. The time at which the mixture attained a stable visual appearance was determined by visual observation and recorded as the particle dissolution time.

For reference, a particle consisting of 100% by weight polyethylene glycol having a weight average molecular weight of 9000 had a particle dissolution time of 11 minutes. Further, for reference, a particle consisting of 100% stearyl amidopropyl dimethyalmine did not attain a stable visual appearance after more than 60 minutes.

Table 1 lists the particle dissolution time for various prepared specimens of particles. Particle dissolution times reported as greater than some time indicate that a stable visual appearance was not achieved within such time.

**Table 1. Particle dissolution time in a solution containing detergent composition and particles consisting of the listed weight percent of fatty amine, fatty acid, dispersing aid, cationic polymer, and balance polyethylene glycol having a weight average molecular weight of 9000.**

| Fatty Amine | Weight Percent of Fatty Amine | Fatty Acid | Weight Percent of Fatty Acid | Dispersing Aid¹ | Weight Percent Cationic Polymer² | Molar Ratio of Fatty Amine: Fatty Acid | Particle Dissolution Time (minutes) |
|---|---|---|---|---|---|---|---|
| stearyl amidopropyl dimethyalmine | 100 | none | 0 | 0 | 0 | - | >60 |
| stearyl amidopropyl dimethyalmine | 19 | none | 0 | 0 | 3 | - | >40 |
| stearyl amidopropyl dimethyalmine | 19 | C₁₂₋₁₈ fatty acid | 11 | 0 | 3 | 1:1 | 36 |
| stearyl amidopropyl dimethyalmine | 19 | cumene sulfononic acid | 11 | 7.5 | 3 | 1:1 | 33 |
| stearyl amidopropyl dimethyalmine | 17 | dodecenyl succinic acid | 13 | 7.5 | 3 | 1:1 | 14 |
| stearyl amidopropyl dimethyalmine | 17 | oleic acid | 13 | 0 | 3 | 1:1 | 22 |
| stearyl amidopropyl dimethyalmine | 23 | oleic acid | 7 | 0 | 3 | 2.3:1 | 28 |
| stearyl amidopropyl dimethyalmine | 19 | myristic acid | 11 | 0 | 3 | 1:1 | 29 |
| stearyl amidopropyl dimethyalmine | 19 | lauric acid | 11 | 0 | 3 | 1:1 | 19 |
| N,N-bis(tallowyl-oxy-ethyl) N-methyl amine | 21 | none | 0 | 0 | 3 | - | >40 |
| N,N-bis(tallowyl-oxy-ethyl) N-methyl amine | 23 | cumene sulfonic acid | 7 | 0 | 3 | 1:1 | 33 |
| N,N-bis(tallowyl-oxy-ethyl) N-methyl amine | 23 | C₁₂₋₁₈ fatty acid | 7 | 0 | 3 | 1:1 | 36 |
| N,N-bis(tallowyl-oxy-ethyl) N-methyl amine | 21 | oleic acid | 9 | 0 | 3 | 1:1 | 14 |

| | | | | | | | |
|---|---|---|---|---|---|---|---|
| ¹ SURFONIC TDA-11 branched isotridecyl alcohol having an average of 11 EO units, available from Huntsman Corp, Salt Lake City, UT. ² Cationic hydroxyethyl cellulose having a weight average molecular weight of 400 kDa, a charge density of 0.18, and an average weight percent of nitrogen per anydroglucose repeat unit of 0.28% (Polymer PK available from Dow Chemical). | | | | | | | |

As shown in Table 1, including a fatty acid in the particles tends to reduce the particle dissolution time. A short dissolution time can provide for the benefit of complete particle dissolution during a treatment or wash cycle so that remnants of the particles are not present at the end of the treatment or wash cycle. Further, a short dissolution time can also provide for the benefit of completely releasing the fabric softening active or actives to the wash liquor to be available for deposition onto the laundry being treated.

Table 2 lists the particle dissolution time for various prepared specimens of particles.

**Table 2. Particle dissolution time in a solution containing detergent composition and particles consisting of the listed weight percent of fatty amine, fatty acid, dispersing aid, 3 % by weight cationic polymer¹, and 67% by weight polyethylene glycol having a weight average molecular weight of 9000. The second and ninth rows (counting the table heading row) are duplicates of results listed in Table 1.**

| Fatty Amine | Weight Percent of Fatty Amine | Fatty Acid | Weight Percent of Fatty Acid | Dispersing Aid | Dispersing Aid Physical Form | Weight Percent Dispersing Aid | Molar Ratio of Fatty Amine: Fatty Acid | Particle Dissolution Time (minutes) |
|---|---|---|---|---|---|---|---|---|
| stearyl amidopropyl dimethyalmine | 19 | C₁₂₋₁₈ fatty acid | 11 | - | - | 0 | 1:1 | 36 |
| stearyl amidopropyl dimethyalmine | 19 | C₁₂₋₁₈ fatty acid | 11 | SURFONIC TDA-11² | Liquid | 7.5 | 1:1 | 26 |
| stearyl amidopropyl dimethyalmine | 19 | C₁₂₋₁₈ fatty acid | 11 | Sorbitol E4 C120³ | Liquid | 7.5 | 1:1 | 32 |
| stearyl amidopropyl dimethyalmine | 19 | C₁₂₋₁₈ fatty acid | 11 | PEI 600-E10⁴ | Liquid | 7.5 | 1:1 | 22 |
| stearyl amidopropyl dimethyalmine | 14 | C₁₂₋₁₈ fatty acid | 16 | - | - | 0 | 0.5:1 | >40 |
| stearyl amidopropyl dimethyalmine | 14 | C₁₂₋₁₈ fatty acid | 16 | SURFONIC TDA-11² | Liquid | 7.5 | 0.5:1 | 27 |
| stearyl amidopropyl dimethyalmine | 14 | C₁₂₋₁₈ fatty acid | 16 | PEI 600-E30⁴ | Solid | 7.5 | 0.5:1 | 34 |
| N,N-bis(tallowyl-oxy-ethyl) N-methyl amine | 23 | cumene sulfonic acid | 7 | - | - | 0 | 1:1 | 33 |
| N,N-bis(tallowyl-oxy-ethyl) N-methyl amine | 23 | cumene sulfonic acid | 7 | SURFONIC TDA-11² | Liquid | 7.5 | 1:1 | 18 |
| N,N-bis(tallowyl-oxy-ethyl) N-methyl amine | 23 | cumene sulfonic acid | 7 | NEODOL 25-9⁵ | Solid | 7.5 | 1:1 | 38 |

| | | | | | | | | |
|---|---|---|---|---|---|---|---|---|
| ¹ Cationic hydroxyethyl cellulose having a weight average molecular weight of 400 kDa, a charge density of 0.18, and an average weight percent of nitrogen per anydroglucose repeat unit of 0.28% (Polymer PK available from Dow Chemical). ² SURFONIC TDA-11 branched isotridecyl alcohol with average of 11 EO units, available from Huntsman Corp, Salt Lake City, UT. ³ Ethoxylated sorbitol capped with a alkyl group of 12 carbon atoms. ⁴ Polyethylene imine of molecular weight 600 with 10 moles of ethoxylation per nitrogen. ⁵ C12-15 alcohol ethoxylated with 9 moles of EO, available from Shell Chemicals, Houston, TX. | | | | | | | | |

As shown in Table 2, including a liquid dispersing aid tends to reduce the particle dissolution time, with short dissolution times providing a benefit as described above with respect to Table 1

To evaluate the efficacy of various fatty amine compounds for delivering a fabric softening benefit, the battery of tests listed in Table 3 was performed. North America Kenmore 600 Series top-loading washing machines were used. Each machine was set to run a Normal single cycle including a 12 minute wash agitation period, and 1 three-minute rinse. The water used was 137 ppm hardness and 25 °C for the wash, and 15.5 °C for the rinse. The water volume at each step was 64 Liters. The total fabric load weight was 3.6 kg (which included 10 test fabric hand towel terry cloths, about 1.3kg of 100% cotton T-shirts, about 800g of 100% cotton hand towels, and about 300g of 50/50 polycotton T-shirt pieces. The detergent used was TIDE Original Scent liquid without perfume (produced by The Procter & Gamble Company). 81 g of detergent was dosed into the wash water while the wash water was filling. After the detergent was added, 30.8 g of the particles being evaluated were also added, followed by the fabric load. After the water fill was complete, the machine entered the agitation period. This was followed by the wash agitation (Normal setting), and the rinse step (with corresponding spin cycle). After the wash process was completed, the fabrics were removed. The test fabrics were machine dried in Kenmore driers on Cotton/High setting, for 50 minutes. The test fabrics were then equilibrated for 24 hours in a 70F / 50% Relative Humidity controlled room. After the test fabric terry cloths had equilibrated, the coefficient of friction of each terry was evaluated. The kinematic coefficient of friction was measured using a Thwing Albert Friction/Peel Tester FP-2250 by attaching a swatch cut from the terry cloth to a sled and dragging the sled over a portion of the remaining terry cloth at a fixed rate. The softness benefit reported in Table 3 is the decrease in the coefficient of friction relative to a detergent only control. The larger the softness benefit, the lower the coefficient of friction of the fabric as compared to the detergent only control. The average for the 10 terry cloths washed in the respective product are reported in Table 3.

**Table 3. Softness benefit provided by particles consisting of the listed weight percent of fatty amine, fatty acid, cationic polymer, and polyethylene glycol (PEG) having a weight average molecular weight of 9000.**

| Fatty Amine | Weight Percent of Fatty Amine | Fatty Acid | Weight Percent of Fatty Acid | Weight Percent of Cationic Polymer¹ | Weight Percent of PEG | Softness Benefit |
|---|---|---|---|---|---|---|
| stearyl amidopropyldim ethyalmine | 19 | C12-18 fatty acid | 11 | 3 | 67 | 0.33 |
| behenyl amidopropyl dimethylamine | 24 | cumene sulfonic acid | 6 | 3 | 67 | 0.33 |
| N,N-bis(tallowyl-oxy-ethyl) N-methyl amine | 23 | cumene sulfonic acid | 7 | 3 | 67 | 0.27 |
| N,N-bis(tallowyl-oxy-ethyl) N-hydroxyethyl amine | 23 | cumene sulfonic acid | 7 | 3 | 67 | 0.22 |
| ditallowamine | 23 | cumene sulfonic acid/sulfu ric acid (7.5/1) | 7 | 3 | 67 | 0.45 |
| stearylamine | 15 | stearic acid | 15 | 4 | 66 | 0.38 |
| tristearylamine | 25 | cumene sulfonic acid/Sulf uric acid (7.5/1) | 5 | 3 | 67 | 0.39 |

| | | | | | | |
|---|---|---|---|---|---|---|
| ¹ Cationic hydroxyethyl cellulose having a weight average molecular weight of 400 kDa, a charge density of 0.18, and an average weight percent of nitrogen per anydroglucose repeat unit of 0.28% (Polymer PK available from Dow Chemical). | | | | | | |

As shown in Table 3, each of the compositions evaluated provide a softness benefit as compared to the detergent only control.

### Combinations

An example is below:
A. A composition comprising a plurality of particles, said plurality of particles comprising:
   about 25% to about 94% by weight a water soluble or water dispersible carrier;
   about 0.5% to about 10% by weight a cationic polymer; and
   about 5% to about 45% by weight a fatty amine of the structure 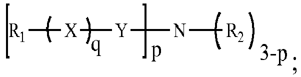
      wherein each R₁ is independently selected from the group consisting of C₈-C₃₂ alkyl, C₈-C₃₂ substituted alkyl, C₆-C₃₂ aryl, C₅-C₃₂ substituted aryl, C₆-C₃₂ alkylaryl, C₆-C₃₂ substituted alkylaryl;
      X is a 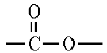 group or a 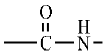 group;
      Y is an alkylene radical having 1-6 carbon atoms;
      N is a nitrogen atom;
      R₂ is independently selected from the group consisting of H, C₁-C₆ alkyl, hyroxyalkyl and polyhydroxyalkyl;
      q is 0 or 1;
      p is an integer from 1-3; and
      wherein said plurality of particles comprises individual particles, wherein said individual particles each have a mass from about 1 mg to about 1 g.
   B. The composition according to Paragraph A, wherein
      each R₁ is independently C₁₀-C₂₂ alkyl or C₈-C₂₂ substituted alkyl;
      X is said 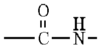 group;
      Y is an alkylene radical having 2-4 carbon atoms; and
      R₂ is independently H or C₁-C₆ alkyl;
      q is 1; and
      p is 1.
   C. The composition according to Paragraph B, wherein each R₁ is independently C₁₀-C₂₂ alkyl and R₂ is a methyl group.
   D. The composition according to Paragraph C, wherein said fatty amine is of the structure 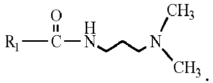
   E. The composition according to Paragraph A, wherein
      R₁ is a C₁₂-C₂₂ alkyl;
      X is a 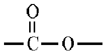 group;
      Y is an alkylene radical with 2-4 carbon atoms;
      R₂ is independently selected from the group consisting of H, C₁-C₆ alkyl, hyroxyalkyl and polyhydroxyalkyl groups;
      q=1; and
      p=1 to 3.
   F. The composition according to Paragraph E, wherein said fatty amine is of a structure selected from the group consisting of: 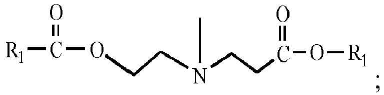 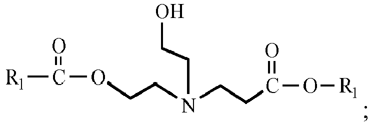 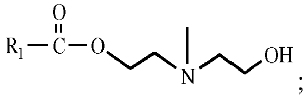 and 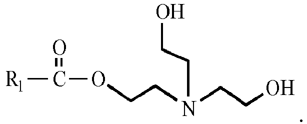
   G. The composition according to Paragraph A, wherein
      R₁ is a C₁₀-C₁₈ alkyl;
      Y is an alkylene radical having 2-4 carbon atoms;
      R₂ is independently selected from the group consisting of H, C₁-C₆ alkyl, hyroxyalkyl and polyhydroxyalkyl groups;
      q=0; and
      p= 1-3.
   H. The composition according to Paragraph A, wherein said fatty amine selected from the group consisting of: esters of bis-(2-hydroxypropyl)-methylamine, bis-(hydroxyethyl)-methyl amine and triethanolamine with at least one fatty acid comprising C₁₂-C₂₂ alkyl chain; N, N- bis(stearoyl-oxy-ethyl)-N-hydroxyethylamine; N,N-bis-(stearoyl-2-hydroxypropyl)-N-methylamine; N,N-bis(stearoyl-oxy-ethyl)-N-methyl amine; N, N-bis(stearoyl-oxy-ethyl)-N-hydroxyethylamine; N-(stearoyl-2-hydroxypropyl)-N,N-dimethylamine; N-(stearoyl-oxy-ethyl)-N, N-dimethyl amine; N-(stearoyl-oxy-ethyl)-N, N-hydroxyethylamine; N,N,N-tris(stearoyl-oxy-ethyl)-amine; stearylamidoproyl dimethyl amine; cocoamidopropyl domethylamine; behenylamidopropyl dimentylamine; stearylamine; distearylamine tristearylamine; and N, N-distearyl N-methylamine.
   I. The composition according to any of Paragraphs A to H, wherein said plurality of particles further comprises fatty acid at a molar ratio of said fatty amine to said fatty acid from about 1:3 to about 3:1.
   J. The composition according to any of Paragraph I, wherein said fatty acid is selected from the group consisting of stearic acid, aryl sulfonic acid, cumenesulfonic acid, xylene sulfonic acid, toluene sulfonic acid, alkenyl succinic acid, octyl succinic acid, dodecenylsuccinic acid, C₁₂-C₁₈ fatty acid, oleic acid, lauric acid myristic acid, and combinations thereof.
   K. The composition according to any of Paragraphs A to J, wherein said fatty amine is selected from the group consisting of dimethylamidopropyl stearamide, esters of bis-(2-hydroxypropyl)-methylamine, bis-(hydroxyethyl)-methyl amine, bis-(hydroxyethyl)-isopropyl amine and triethanolamine with at least one fatty acid comprising C₁₂-C₂₂ alkyl chain; N,N-bis(stearoyl-oxy-ethyl)-N-hydroxyethylamine; N,N-bis-(stearoyl-2-hydroxypropyl)-N-methylamine; N,N-bis(stearoyl-oxy-ethyl)-N-methyl amine; N, N-bis(stearoyl-oxy-ethyl)-N-hydroxyethylamine; N-(stearoyl-2-hydroxypropyl)-N,N-dimethylamine; N-(stearoyl-oxy-ethyl)-N, N-dimethyl amine; N-(stearoyl-oxy-ethyl)-N, N-hydroxyethylamine; N,N,N-tris(stearoyl-oxy-ethyl)-amine; and combinations thereof.
   L. The composition according to any of Paragraphs A to K, wherein said cationic polymer is a cationic polysaccharide.
   M. The composition according to Paragraph L, wherein said cationic polysaccharide is polymeric quaternary ammonium salt of hydroxyethylcellulose which has been reacted with an epoxide substituted with a trimethylammonium group.
   N. The composition according to any of Paragraphs A to M, wherein said particles further comprise a dispersing aid.
   O. The composition according to any of Paragraphs A to N, wherein said fatty amine and said cationic polymer are dispersed in said carrier.
   P. The composition according to any of Paragraphs A to O, wherein said carrier is a water soluble polymer.
   Q. The composition according to any of Paragraphs A to P, wherein said carrier is selected from the group consisting of:
      a polyalkylene polymer of formula H-(C₂H₄O)ₓ-(CH(CH₃)CH₂O)_{y}-(C₂H₄O)_{z}-OH wherein x is from about 50 to about 300, y is from about 20 to about 100, and z is from about 10 to about 200;
      a polyethylene glycol fatty acid ester of formula (C₂H₄O)_{q}-C(O)O-(CH₂)ᵣ-CH₃ wherein q is from about 20 to about 200 and r is from about 10 to about 30;
      a polyethylene glycol fatty alcohol ether of formula HO-(C₂H₄O)ₛ-(CH₂)ₜ)-CH₃ wherein s is from about 30 to about 250 and t is from about 10 to about 30;
      C8-C22 alkyl polyalkoxylate comprising more than about 40 alkoxylate units;
      polyethylene glycol having a weight average molecular weight from about 2000 to about 15000;
      EO/PO/EO block copolymer;
      PO/EO/PO block copolymer;
      EO/PO block copolymer;
      PO/EO block copolymer;
      polypropylene glycol;
      ethoxylated nonionic surfactant having a degree of ethoxylation greater than about 30;
      polyvinyl alcohol;
      polyalkylene glycol having a weight average molecular weight from about 2000 to about 15000;
      and mixtures thereof.
   R. The composition according to any of Paragraphs A to Q, wherein said plurality of particles further comprise about 5% to about 25% by weight fatty acid, wherein said carrier is a water soluble polymer, wherein said cationic polymer is a cationic polysaccharide, wherein said plurality of particles have an onset of melt from about 25 °C to about 120 °C, and wherein said fatty amine, said cationic polymer, and said fatty acid are dispersed in said carrier.
   S. A process for treating an article of clothing comprising the steps of:
      providing an article of clothing in a washing machine; and
      contacting said article of clothing during a wash sub-cycle of said washing machine with the composition according to any of Paragraphs A to R.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A composition comprising a plurality of particles, said plurality of particles comprising:
25% to 94% by weight a water soluble or water dispersible carrier;
0.5% to 10% by weight a cationic polymer; and
5% to 45% by weight a fatty amine of the structure 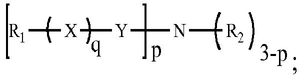
wherein each R₁ is independently selected from the group consisting of C₈-C₃₂ alkyl, C₈-C₃₂ substituted alkyl, C₆-C₃₂ aryl, C₅-C₃₂ substituted aryl, C₆-C₃₂ alkylaryl, C₆-C₃₂ substituted alkylaryl;
X is a 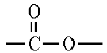 group or a 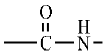 group;
Y is an alkylene radical having 1-6 carbon atoms;
N is a nitrogen atom;
R₂ is independently selected from the group consisting of H, C₁-C₆ alkyl, hyroxyalkyl and polyhydroxyalkyl;
q is 0 or 1;
p is an integer from 1-3; and
wherein said plurality of particles comprises individual particles, wherein said individual particles each have a mass from 1 mg to 1 g.

2. The composition according to Claim 1, wherein
each R₁ is independently C₁₀-C₂₂ alkyl or C₈-C₂₂ substituted alkyl;
X is said 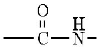 group;
Y is an alkylene radical having 2-4 carbon atoms; and
R₂ is independently H or C₁-C₆ alkyl;
q is 1; and
p is 1.

3. The composition according to Claim 2, wherein each R₁ is independently C₁₀-C₂₂ alkyl and R₂ is a methyl group.

4. The composition according to Claim 3, wherein said fatty amine is of the structure

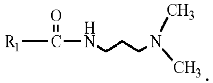

5. The composition according to Claim 1, wherein
R₁ is a C₁₂-C₂₂ alkyl;
X is a 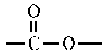 group;
Y is an alkylene radical with 2-4 carbon atoms;
R₂ is independently selected from the group consisting of H, C₁-C₆ alkyl, hyroxyalkyl and polyhydroxyalkyl groups;
q=1; and
p=1 to 3.

6. The composition according to Claim 5, wherein said fatty amine is of a structure selected from the group consisting of: 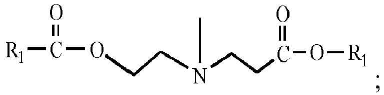 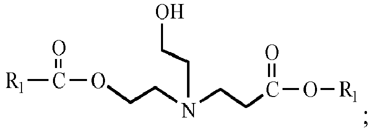 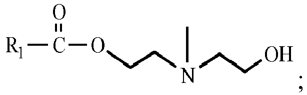 and

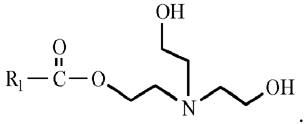

7. The composition according to Claim 1, wherein
R₁ is a C₁₀-C₁₈ alkyl;
Y is an alkylene radical having 2-4 carbon atoms;
R₂ is independently selected from the group consisting of H, C₁-C₆ alkyl, hyroxyalkyl and polyhydroxyalkyl groups;
q=0; and
p=1-3.

8. The composition according to Claim 1, wherein said fatty amine selected from the group consisting of: esters of bis-(2-hydroxypropyl)-methylamine, bis-(hydroxyethyl)-methyl amine and triethanolamine with at least one fatty acid comprising C₁₂-C₂₂ alkyl chain; N, N-bis(stearoyl-oxy-ethyl)-N-hydroxyethylamine; N,N-bis-(stearoyl-2-hydroxypropyl)-N-methylamine; N,N-bis(stearoyl-oxy-ethyl)-N-methyl amine; N, N- bis(stearoyl-oxy-ethyl)-N-hydroxyethylamine; N-(stearoyl-2-hydroxypropyl)-N,N-dimethylamine; N-(stearoyl-oxy-ethyl)-N, N-dimethyl amine; N-(stearoyl-oxy-ethyl)-N, N-hydroxyethylamine; N,N,N-tris(stearoyl-oxy-ethyl)-amine; stearylamidoproyl dimethyl amine; cocoamidopropyl domethylamine; behenylamidopropyl dimentylamine; stearylamine; distearylamine tristearylamine; and N, N-distearyl N-methylamine.

9. The composition according to any of the preceding claims, wherein said plurality of particles further comprises fatty acid at a molar ratio of said fatty amine to said fatty acid from 1:3 to 3:1.

10. The composition according to Claim 9, wherein said fatty acid is selected from the group consisting of stearic acid, aryl sulfonic acid, cumenesulfonic acid, xylene sulfonic acid, toluene sulfonic acid, alkenyl succinic acid, octyl succinic acid, dodecenylsuccinic acid, C₁₂-C₁₈ fatty acid, oleic acid, lauric acid myristic acid, and combinations thereof.

11. The composition according to any of the preceding claims, wherein said fatty amine is selected from the group consisting of dimethylamidopropyl stearamide, esters of bis-(2-hydroxypropyl)-methylamine, bis-(hydroxyethyl)-methyl amine, bis-(hydroxyethyl)-isopropyl amine and triethanolamine with at least one fatty acid comprising C₁₂-C₂₂ alkyl chain; N, N-bis(stearoyl-oxy-ethyl)-N-hydroxyethylamine; N,N-bis-(stearoyl-2-hydroxypropyl)-N-methylamine; N,N-bis(stearoyl-oxy-ethyl)-N-methyl amine; N, N- bis(stearoyl-oxy-ethyl)-N-hydroxyethylamine; N-(stearoyl-2-hydroxypropyl)-N,N-dimethylamine; N-(stearoyl-oxy-ethyl)-N, N-dimethyl amine; N-(stearoyl-oxy-ethyl)-N, N-hydroxyethylamine; N,N,N-tris(stearoyl-oxy-ethyl)-amine; and combinations thereof.

12. The composition according to any of the preceding claims, wherein said cationic polymer is a cationic polysaccharide, preferably polymeric quaternary ammonium salt of hydroxyethylcellulose which has been reacted with an epoxide substituted with a trimethylammonium group.

13. The composition according to any of the preceding claims, wherein said carrier is selected from the group consisting of:
a polyalkylene polymer of formula H-(C₂H₄O)ₓ-(CH(CH₃)CH₂O)_{y}-(C₂H₄O)_{z}-OH wherein x is from 50 to 300, y is from 20 to 100, and z is from 10 to 200;
a polyethylene glycol fatty acid ester of formula (C₂H₄O)_{q}-C(O)O-(CH₂)ᵣ-CH₃ wherein q is from 20 to 200 and r is from 10 to 30;
a polyethylene glycol fatty alcohol ether of formula HO-(C₂H₄O)ₛ-(CH₂)ₜ)-CH₃ wherein s is from 30 to 250 and t is from 10 to 30;
C8-C22 alkyl polyalkoxylate comprising more than 40 alkoxylate units;
polyethylene glycol having a weight average molecular weight from 2000 to 15000;
EO/PO/EO block copolymer;
PO/EO/PO block copolymer;
EO/PO block copolymer;
PO/EO block copolymer;
polypropylene glycol;
ethoxylated nonionic surfactant having a degree of ethoxylation greater than 30;
polyvinyl alcohol;
polyalkylene glycol having a weight average molecular weight from 2000 to 15000;
and mixtures thereof.

14. The composition according to any of the preceding claims, wherein said plurality of particles further comprises 5% to 25% by weight fatty acid, wherein said carrier is a water soluble polymer, wherein said cationic polymer is a cationic polysaccharide, wherein said plurality of particles have an onset of melt from 25 °C to 120 °C, and wherein said fatty amine, said cationic polymer, and said fatty acid are dispersed in said carrier.

15. A process for treating an article of clothing comprising the steps of:
providing an article of clothing in a washing machine; and
contacting said article of clothing during a wash sub-cycle of said washing machine with the composition according to any of the preceding claims.
